# EUROPÄISCHE PATENTSCHRIFT

(11) **EP 1 038 542 B1**
(45) Veröffentlichungstag und Bekanntmachung des Hinweises auf die Patenterteilung: **03.03.2004**
(21) Anmeldenummer: 00105939.3
(22) Anmeldetag: 23.03.2000
(51) Int. Cl.: A61M 5/30

(54) **Nadelloses Injektionsgerät**
Needleless injection device
Dispositif d'injection sans aiguille

(30) Priorität: 24.03.1999 DE 19913344
(43) Veröffentlichungstag der Anmeldung: 27.09.2000
(73) Patentinhaber: Deutscher Zahnarzt Verlag (DZV), 92224 Amberg (DE)
(72) Erfinder: Golan, Ygal, 92224 Amberg (DE)
(74) Vertreter: VOSSIUS & PARTNER

(56) Entgegenhaltungen:
- EP-A- 0 286 798
- EP-A- 0 370 571
- WO-A-91/12839
- WO-A-99/07435
- US-A- 2 675 802
- US-A- 5 569 034

## Beschreibung

Die vorliegende Erfindung betrifft ein nadelloses Injektionsgerät zum Injizieren von Flüssigkeiten in den Körper eines Patienten, insbesondere im oralen Bereich.

Für viele Patienten ist der unangenehmste Augenblick eines Zahnarztbesuchs die Angst vor dem Nadeleinstich zur Applikation eines Lokalanästhetikums. Für die unterschiedlichen Formen der Lokalanästhesie werden verschiedene Typen von Injektionsgeräten angeboten. So werden in der Leitungs- und der Infiltrationsanästhesie Injektionsspritzen benutzt, mit denen aus Sicherheitsgründen eine Aspiration vor jeder Injektion möglich ist. Bei den Einmal-Kunststoff- oder Glaskolbenspritzen kann dies durch einfaches Zurückziehen des Spritzenkolbens erreicht werden. Bei Zylinderampullenspritzen liegt ein spezieller Klemmechanismus vor, mit dem ein Gummistempel zurückgezogen werden kann. Durch Kanülen unterschiedlicher Stärke gelangt das Lokalanästhetikum in die entsprechende Geweberegion. Während früher fast ausschließlich sterilisierbare Stahlkanülen verwendet wurden, haben sich mittlerweile die sterilisierten Einmalkanülen durchgesetzt. Diese konventionellen Spritzen haben allesamt den Nachteil, daß zur Injektion des Lokalanästhetikums eine Nadel bzw. Kanüle erforderlich ist, die beim Patienten einen erheblichen Injektionsschmerz verursacht.

Aus der WO-A-96/36381 ist beispielsweise ein Injektionsgerät zur Hochdruckinjektion einer Flüssigkeit oder einer Partikel enthaltenden Flüssigkeit bekannt. Dieses Injektionsgerät weist eine Druckkammer auf, die in eine Injektionsöffnung mündet und die von einem Arbeitskolben begrenzt wird, der von einem Antrieb in der Druckkammer verlagerbar ist. Der Antrieb ist als ein einen elastischen Stoß auf den Arbeitskolben ausübendes Anschlagstück ausgebildet, das angetrieben bis zum Anschlag an dem der Druckkammer abgewandten Ende des Arbeitskolbens beschleunigbar ist. Dabei ist das Anschlagstück mit der Stoßübertragung antriebslos und das Volumen der Druckkammer ist größer als das Verdrängungsvolumen der von dem Arbeitskolben bei dessen Arbeitsschub verdrängten Flüssigkeit.

Weitere nadellose Injektionsgeräte sind beispielsweise aus der WO-A-94/07554, WO-A-95/03844, WO-A-96/24398, WO-A-95/27523, WO-A-95/16481, WO-A-97/13536, EP-A-370 571, EP-A-286 798, EP-A-276 158 oder DE-A-33 28 173 bekannt.

EP-A-286 798 wird als nächstkommender Stand der Technik angesehen. Die bekannten nadellosen Injektionsgeräte haben insbesondere den Nachteil, daß sie aufgrund des Lademechanismus relativ groß sind und zur Verringerung des Rückstoßes eine relativ große Masse aufweisen. So hat ein auf dem Markt befindliches Injektionsgerät ein Gewicht von ca. 590g, ist etwa 28 cm lang und kostet etwa DM 3,500.00. Ein derartiges Gerät hat allein aufgrund seiner Größe und seines Gewichts für den Patienten psychologische und für den behandelnden Arzt medizinische Nachteile.

Der vorliegenden Erfindung liegt die Aufgabe zugrunde, ein verbessertes nadelloses Injektionsgerät zur Verfügung zu stellen. Diese Aufgabe wird mit den Merkmalen der Ansprüche gelöst.

Dabei geht die Erfindung von dem Grundgedanken aus, daß das nadellose Injektionsgerät einen Kupplungsabschnitt an dem der Düse gegenüberliegenden Ende aufweist, der den an den üblichen zahnärztlichen Winkelwerkzeugen ausgebildeten Kupplungselementen zum Antreiben von Bohrern, Fräsern u.s.w. entspricht. Dies bedeutet, daß das erfindungsgemäße nadellose Injektionsgerät als Lademechanismus für die Aktivierung der Spritze an die zur Grundausstattung eines jeden zahnärztlichen Behandlungsstuhls gehörende Kupplung für die dort vorgesehenen Behandlungswerkzeuge ankoppelbar ist. Dazu ist vorzugsweise an dem der Düse gegenüberliegenden Endabschnitt des Injektionsgeräts ein Rotationselement vorgesehen, das mit einem Zahnrad und einem weiteren axial beweglichen Rotationselement zur Vorspannung eines Federelements in Verbindung steht.

Das erfindungsgemäße nadellose Injektionsgerät hat gegenüber bekannten Injektionsgeräten insbesondere die Vorteile, daß kein komplizierter Lademechanismus zum Vorspannen eines Federelements erforderlich ist und daß das Gerät selbst mit relativ geringer Baugröße und geringem Gewicht ausgebildet werden kann, da es mit der Antriebseinrichtung über einen Verbindungskanal verbunden ist, der eine ausreichende Masse zur Verminderung des Rückstoßes aufweist. Ferner ist das erfindungsgemäße nadellose Injektionsgerät aus wenigen Teilen aufgebaut, wartungsarm bzw. -frei und leicht sterilisierbar. Darüber hinaus können handelsübliche Karpulen für die zu injizierende Flüssigkeit weiterhin verwendet werden. Gegenüber Injektionsgeräten mit Kanülen besteht keine Gefahr des Kanülenbruchs und das Risiko von Allergien und Intoxikationen kann durch die geringere Injektionsmenge erheblich verringert werden. Ein weiterer wesentlicher Vorteil des erfindungsgemäßen Injektionsgeräts ist, daß es nadellos funktioniert, d.h. die Patienten müssen keine Angst mehr vor schmerzhaften Nadeleinstichen haben. Vielmehr ist bei der Injektion eines Lokalanästhetikums mit dem erfindungsgemäßen Injektionsgerät lediglich ein einmaliges kurzes Klopfen auf dem Zahnfleisch zu spüren. Dadurch wird die Akzeptanz dieser Injektionsmethode weiter gesteigert, so daß bessere zahnärztliche und oralchirurgische Behandlungserfolge zu erwarten sind.

Das erfindungsgemäße nadellose Injektionsgerät wird nachstehend anhand einer bevorzugten Ausführungsform unter Bezugnahme auf die Zeichnung beschrieben. Es zeigt:

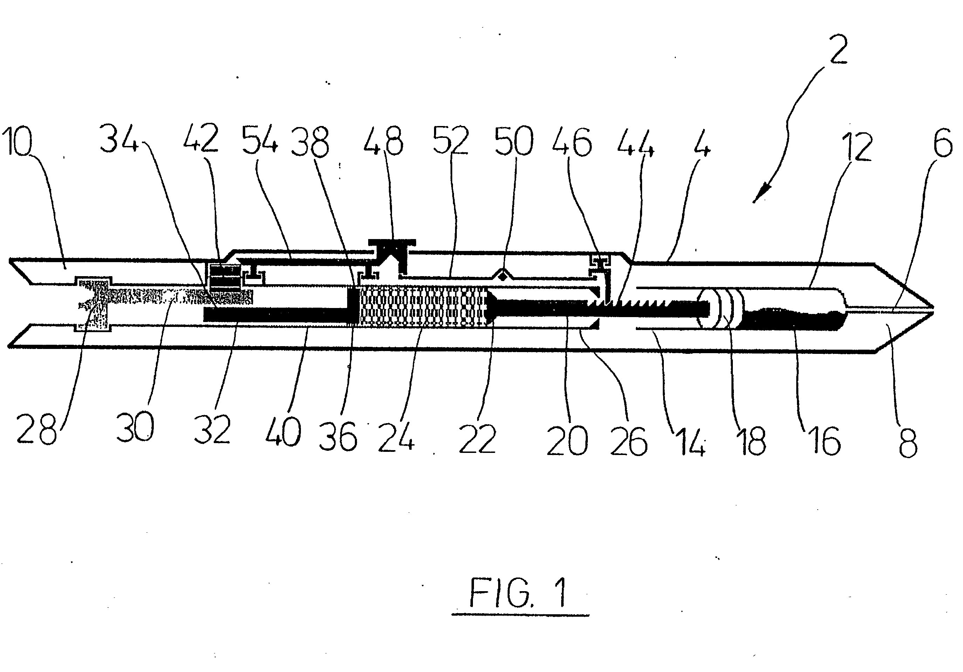

- Fig. 1: einen schematischen Längssschnitt durch das erfindungsgemäße nadellose Injektionsgerät.

Das in Fig. 1 dargestellte erfindungsgemäße nadellose Injektionsgerät 2 weist ein vorzugsweise im wesentlichen zylindrisches Gehäuse 4 mit einem ersten eine Auslaßdüse 6 aufweisenden Ende 8 und einem zweiten der Düse gegenüberliegenden Ende 10 auf. Das Gehäuse ist vorzugsweise aus Metall oder einer Metallegierung hergestellt, kann jedoch auch aus Kunststoff, Glas und sonstigen leicht sterilisierbaren Materialien gefertigt sein.

Die im ersten Ende 8 des Gehäuses 4 vorgesehene Düse 6 mündet im Gehäuse in einen Karpulenaufnahmeabschnitt 14 zur Aufnahme handelsüblicher Karpulen 12, die das zu injizierende Fluid 16 enthalten. Zum Hochdruckinjizieren des Fluids ist im Gehäuse 4 ferner ein axial beweglicher Kolben 18 vorgesehen, der in die Karpule 12 hinein beweglich und darin abgedichtet geführt ist. Wird der Kolben 18 schnell in der Karpule 12 in Richtung der Düse 6 bewegt, so wird das zu injizierende Fluid 16 unter hohem Druck aus dem erfindungsgemäßen nadellosen Injektionsgerät 2 abgegeben und dringt abhängig vom Druck in den Körper eines Patienten ein.

An einer der Düse 6 gegenüberliegenden Seite des Kolbens 18 ist vorzugsweise eine Kolbenstange 20 vorgesehen, die mit einem Federteller 22 mit einem Federelement 24 in Wirkverbindung steht. Die Kolbenstange 20 ist für das erfindungsgemäße nadellose Injektionsgerät 2 lediglich bevorzugt, es ist jedoch auch möglich, daß das Federelement 24 direkt auf die von der Düse 6 abgewandte Seite des Kolbens 18 wirkt. Das Federelement 24 ist vorzugsweise eine Druckfeder, wie z.B. eine Spiraldruckfeder oder ein Tellerfedern-Paket, eine Gasdruckfeder, ein hydraulisches Federelement oder ein ein kompressibles Medium enthaltender Volumenraum.

Das Federelement 24 ist vorzugsweise in einer im Gehäuse 4 vorgesehenen Führungseinrichtung 26 axial beweglich gelagert. Das in Fig. 1 dargestellte nadellose Injektionsgerät 2 ist mit vorgespanntem Federelement 24 dargestellt. Zum Vorspannen des Federelements 24 ist ein Spannmechanismus vorgesehen, der mit einer herkömmlichen zahnärztlichen Antriebseinrichtung, wie sie beispielsweise an einem Zahnarztstuhl zum Antreiben von Bohrern u.s.w. vorgesehen ist, verbindbar ist. Dazu ist es bevorzugt, am Ende 10 des Gehäuses 4 ein Kupplungselement 28 zum Ankuppeln der (nicht dargestellten) Antriebseinrichtung vorzusehen. Dieses Kupplungselement 28 stellt einen Teil eines ersten, im wesentlichen axial festgelegten Rotationselements dar. Das Rotationselement weist ein sich in Axialrichtung erstreckendes Verlängerungselement 30 mit vorzugsweise im wesentlichen halbrunden Querschnittsprofil auf. Daneben weist der Lademechanismus ein zweites, axial bewegliches Rotationselement 32 auf, das ebenfalls über das Kupplungselement 28 in Rotationsbewegung versetzbar ist. Auch das zweite Rotationselement 32 weist vorzugsweise einen im wesentlichen halbrunden Querschnitt auf. Die beiden Rotationselemente bilden dann zusammen in einem Überlappungsabschnitt 34 einen im wesentlichen zylindrischen Außenumfang. Dadurch wird bei einer Drehung des ersten Rotationselements 30 das zweite Rotationselement 32 mitbewegt. Das zweite Rotationselement 32 weist einen Axialführungsabschnitt 36 auf, an dem sich das Federelement 24 abstützt. Der Axialführungsabschnitt 36 ist vorzugsweise mit einem Außengewinde 38 versehen, das mit einem im Führungselement 26 vorgesehenen Innengewinde 40 in Eingriff ist. Bei Rotation der Antriebseinrichtung werden die beiden Rotationselemente 30 und 32 in Rotation versetzt, wobei das Gewinde 38 bzw. 40 das zweite Rotationselement entsprechend seiner Steigung axial verschiebt, so daß der Überlappungsabschnitt 34 bei zunehmender Vorspannung des Federelements 24 kleiner wird.

Zur Erzielung einer vorherbestimmbaren bzw. definierten Federvorspannung weist das erfindungsgemäße nadellose Injektionsgerät vorzugsweise eine Begrenzungseinrichtung zum Begrenzen des Axialwegs des zweiten Rotationselements 32 auf. Diese Begrenzungseinrichtung kann beispielsweise durch ein Anschlagmittel für das zweite Rotationselement 32 und eine an der Antriebseinrichtung oder dem Kupplungselement 28 vorgesehene Rutschkupplung ausgebildet sein. Beim Anschlagen des Axialführungselements 36 am Anschlagmittel wird dann die Axialbewegbarkeit des zweiten Rotationselements 32 blockiert, so daß sich auch das erste Rotationselement 30 mit dem Kupplungselement 28 nicht mehr drehen kann. Mit Hilfe der Rutschkupplung wird die von der Antriebseinrichtung zur Verfügung gestellte Rotationsbewegung nicht mehr auf den Spannmechanismus des erfindungsgemäßen Injektionsgeräts 2 übertragen. Alternativ kann die Begrenzungseinrichtung beispielsweise durch ein mit den beiden Rotationselementen 30 und 32 in Eingriff stehendes Zahnrad 42 gebildet sein. Das Zahnrad 42 ist dabei derart ausgebildet, daß es bei einer vorherbestimmbaren Axialposition des zweiten Rotationselements 32 eine weitere Rotation der beiden Rotationselemente verhindert. Dies kann beispielsweise durch entsprechende Ausgestaltung der am Rotationselement 32 vorgesehenen Verzahnung erfolgen. Auch bei dieser Ausführungsform der Begrenzungseinrichtung ist eine Rutschkupplung (nicht dargestellt) an der Antriebseinrichtung oder im Bereich des Kupplungselements 28 vorteilhaft.

Zum Auslösen des erfindungsgemäßen nadellosen Injektionsgeräts 2 muß der Kolben 18 entriegelt werden, um das in der Karpule 12 befindliche Fluid unter hohem Druck durch die Düse 6 auszustoßen. Dazu ist vorzugsweise an der Kolbenstange 20 oder aber auch am Kolben 18 eine Rasteinrichtung 44 vorgesehen, die mit einem am Gehäuse 4 vorgesehenen Rastelement 46 während des Spannens in Eingriff ist. Die Rasteinrichtung 44 kann beispielsweise in Form einer am Kolben 18 oder an der Kolbenstange 20 ausgebildeten Längsverzahnung, in die das Rastelement 46 einrastet, ausgeführt sein. Zum Auslösen des erfindungsgemäßen nadellosen Injektionsgeräts 2 muß das Rastelement 46 außer Eingriff mit der Rasteinrichtung 44 gebracht werden. Dazu ist vorzugsweise ein durch das Gehäuse 4 hindurchragender Auslöseknopf 48 vorgesehen, der bei einem Druck darauf einen über einen Drehpunkt 50 gelagerten Hebelarm 52 verkippt und so den Kolben 18 freigibt. Das Federelement 24 entspannt sich und schiebt den Kolben 18 mittels der Kolbenstange 20 in die handelsübliche Karpule 12 und schießt das darin befindliche Fluid 16 unter hohem Druck aus der Düse 6. Zur Gewährleistung eines sicheren Einrastens des Rastelements 46 in der Rasteinrichtung 44 ist es bevorzugt, ein unter Vorspannung stehendes Federelement vorzusehen.

Ferner kann am erfindungsgemäßen nadellosen Injektionsgerät 2 eine mit dem Auslöseknopf 48 in Wirkverbindung stehende Einrichtung 54 zur Aufhebung der Begrenzungseinrichtung vorgesehen sein. Die Einrichtung 54 ist beispielsweise integral mit dem Auslöseknopf 48 ausgebildet, so daß die Begrenzungseinrichtung freigegeben werden kann.

Das erfindungsgemäße nadellose Injektionsgerät 2 ist vorzugsweise zum Injizieren des Fluids 16 von der Antriebseinrichtung abkoppelbar, so daß ein einfach handhabbares Gerät zur Verfügung steht. Ebenso kann das erfindungsgemäße Gerät 2 jedoch auch mit angekoppelter Antriebseinrichtung verwendet werden.

## Patentansprüche

1. Nadelloses Injektionsgerät (2) zum Injizieren von Fluiden (16) in den Körper eines Patienten mit einem Gehäuse (4), einer an einem ersten Ende (8) vorgesehenen Düse (6), einem eine das zu injizierende Fluid (16) enthaltende Karpule (12) aufnehmenden Karpulenaufnahmeabschnitt (14), einem Kolben (18), einem auf den Kolben (18) wirkendes Federelement (24), einem Spannmechanismus für das Federelement (24) und einem Auslösemechanismus, **dadurch gekennzeichnet, daß** der Spannmechanismus zum Spannen des Federelements (24) mit einer Kupplungseinrichtung einer herkömmlichen zahnärztlichen Antriebseinrichtung verbindbar ist.

2. Injektionsgerät nach Anspruch 1, wobei der Spannmechanismus an einem dem ersten Ende (8) gegenüberliegenden zweiten Ende (10) vorgesehen ist.

3. Injektionsgerät nach Anspruch 1 oder 2, wobei der Spannmechanismus ein Schnellverbindungsmittel (28) zum Verbinden mit der Antriebseinrichtung aufweist.

4. Injektionsgerät nach einem der Ansprüche 1 bis 3, wobei der Spannmechanismus ein erstes axial fixiertes Rotationselement (30) und ein zweites, mit dem ersten Rotationselement (30) rotatorisch in Wirkverbindung stehendes, axial bewegliches Rotationselement (32) aufweist.

5. Injektionsgerät nach Anspruch 4, wobei die Rotationselemente (30, 32) einen im wesentlichen halbrunden Querschnitt aufweisen und gemeinsam im Gehäuse (4) gelagert sind.

6. Injektionsgerät nach Anspruch 4 oder 5, wobei die axiale Beweglichkeit des zweiten Rotationselements (32) durch ein daran vorgesehenes Außengewinde (38), das in Eingriff mit einem im Gehäuse (4) gebildeten Innengewinde (40) ist, ausgebildet ist.

7. Injektionsgerät nach Anspruch 5 oder 6, wobei die beiden Rotationselemente (30, 32) mit einem Verbindungselement miteinander verbunden sind.

8. Injektionsgerät nach einem der Ansprüche 1 bis 7, wobei der Spannmechanismus eine Begrenzungseinrichtung zur definierten Vorspannung des Federelements (24) aufweist.

9. Injektionsgerät nach Anspruch 8, wobei die Begrenzungseinrichtung einen Anschlag und eine Rutschkupplung aufweist.

10. Injektionsgerät nach Anspruch 8, wobei die Begrenzungseinrichtung ein mit den Rotationselementen (30, 32) in Eingriff stehendes Zahnrad (42) ist, das die Axialbewegung des zweiten Rotationselements (32) bei einer vorherbestimmbaren Axialposition blockiert.

11. Injektionsgerät nach Anspruch 10, wobei die Begrenzungseinrichtung ferner eine Rutschkupplung aufweist.

12. Injektionsgerät nach einem der Ansprüche 1 bis 11, wobei der Auslösemechanismus einen Auslöseknopf (48), einen über einen Drehpunkt (50) drehbar gelagerten Auslösehebel (52) und ein mit einer am Kolben (18) oder einer daran vorgesehenen Kolbenstange (20) ausgebildeten Rasteinrichtung (44) in und außer Eingriff bringbares Rastelement (46) aufweist.

13. Injektionsgerät nach Anspruch 12, wobei der Auslösemechanismus eine mit dem Auslöseknopf (48) in Wirkverbindung stehende Einrichtung (54) zur Losung der Begrenzungseinrichtung aufweist.

## Claims

1. Needle-less injecting device (2) to inject fluids (16) into the body of a patient with a housing (4), a nozzle (6) provided on a first end (8), a carpule receiving section (14) designed to receive carpules (12) containing the fluid (16) to be injected, a piston (18), a spring-element (24) acting on the piston (18), and a compressing-mechanism for the spring element (24) and a releasing-mechanism **characterized in that** the compressing-mechanism to tens the spring-element (24) has a coupling unit which can be connected to a customary dental driving unit.

2. Injecting device as claimed in claim 1, wherein the compressing-mechanism is provided on a second end (10) opposite to the first end (8).

3. Injecting device as claimed in claim 1 or 2, wherein the compressing-mechanism comprises a fast connecting-device (28) for connection with the driving unit.

4. Injecting device as claimed in claim 1 to 3, wherein the compressing device comprises a first axially fixed rotation element (30) and a second, axially movable rotation element (32) that is rotatory connected to the first rotation element (30).

5. Injecting device as claimed in claim 4, wherein the rotation elements (30, 32) have an essentially semi-circular cross-section and are embedded together in the housing (4).

6. Injecting device as claimed in claim 4 or 5, wherein the axial mobility of the second rotation-element (32) is realized by a provided external thread (38), meshing with an internal thread (40) formed at the housing (4).

7. Injecting device as claimed in claim 5 or 6, wherein both rotation-elements (30, 32) are connected by a connecting-element.

8. Injecting device as claimed in any one of the claims 1 to 7, wherein the compressing mechanism has a limiting device for achieving a defined bias of the spring element.

9. Injecting device as claimed in claim 8, wherein the limiting device has a stop and a slide coupling.

10. Injecting device as claimed in claim 8, wherein the limiting device is a gearwheel (42) meshing to the rotation elements (30, 32), limiting the axial movement of the second rotation element (32) in a pre-determinable axial position.

11. Injecting device as claimed in claim 10, wherein the limiting device is also equipped with a slide coupling.

12. Injecting device as claimed in any one of claims I to 11, wherein the releasing mechanism has a releasing button (48), a release lever (52) rotatably embedded over a turning point (50), an engagement device (44) arranged at the piston (18) or a piston bar (20) provided thereon and a snap-element (46) that can be brought into and out off contact.

13. Injecting device as claimed in claim 12, wherein the releasing-mechanism has a device (54) connected to the releasing button (48) to release the limiting device.

## Revendications

1. Ustensile d'injection sans aiguille (2) pour injecter des fluides (16) dans le corps d'un patient; avec un boîtier (4), une tuyère (6) sur une première extrémité (8), une section réceptive de seringue (14) pour recevoir une seringue (12) pour le fluide (16) à injecter, , un piston (18), un ressort (24) agissant sur le piston (18), un mécanisme de tension pour le ressort (24) et un système de déclenchement caractérisé que le mécanisme de tension pour tendre le ressort (24) est adaptable au système de propulsion dentaire conventionnel par un embrayage.

2. Ustensile d'injection selon la revendication 1; le mécanisme de tension est prévu sur une première extrémité (8) à l'opposé d'une deuxième extrémité (10).

3. Ustensile d'injection selon la revendication 1 ou 2; le mécanisme de tension comprend un moyen rapide de tension (28) pour raccorder au système de propulsion.

4. Ustensile d'injection selon l'une quelconque des revendications 1 à 3; le mécanisme de tension comprend un premier élément rotatif (30) fixé axialement, et une deuxième élément rotatif (32), axialement mobile en jonction rotative avec le premier élément rotatif (30).

5. Ustensile d'injection selon la revendication 4; les éléments rotatifs (30, 32) sont de préférence semi-circulaires et situés tous les deux dans le boîtier (4).

6. Ustensile d'injection selon la revendications 4 ou 5 ; la mobilité axiale du deuxième élément rotatif (32) est réalisable grâce à un filetage externe (38) fixé auparavant, lui-même engrené à un filetage interne (40) placé dans le boîtier (4).

7. Ustensile d'injection selon la revendication 5 ou 6; les deux éléments rotatifs (30, 32) sont reliés par un élément d'assemblage.

8. Ustensile d'injection selon l'une quelconque des revendications 1 à 7; le mécanisme de tension comprend un dispositif de blocage définir la tension du ressort (24).

9. Ustensile d'injection selon la revendication 8; le dispositif de blocage comprend une butée et un débrayage glissant.

10. Ustensile d'injection selon la revendication 8; le dispositif de blocage est une rue dentée (42) engrenée aux éléments rotatifs (30, 32), qui bloque le mouvement axial du deuxième élément rotatif (32) sur une position prédéterminée sur l'axe.

11. Ustensile d'injection selon la revendication 10; le dispositif de blocage comprend en plus un embrayage glissant.

12. Ustensile d'injection selon l'une quelconque des revendications 1 à 11; le mécanisme de déclenchement comprend un bouton de déclenchement (48), un bras de levier (52) pivotant sur pivot (50) et un élément de suspension (46), à engrener ou non, avec un dispositif de suspension (44) installe soit sur le piston (18), soit sur la tige de piston (20).

13. Ustensile d'injection selon la revendication 12, le dispositif de déclenchement comprend une installation (54) actionnée par le bouton de déclenchement (48) pour débloquer le système de blocage.
